**Europäisches Patentamt**

(19) **European Patent Office**

**Office européen des brevets**

(11) Veröffentlichungsnummer: **0 211 397**
**B1**

(12) **EUROPÄISCHE PATENTSCHRIFT**

(45) Veröffentlichungstag der Patentschrift:
26.10.88

(21) Anmeldenummer: 86110546.8

(22) Anmeldetag: 30.07.86

(51) Int. Cl.⁴: **B 01 J 23/66,** B 01 J 35/10,
C 07 D 301/10

ERRATUM

| | | (SEITE, SPALTE, ZEILE)<br>(PAGE, COLUMN, LINE)<br>(PAGE, COLONNE, LIGNE) | |
|---|---|---|---|

| DIE TEXTSTELLE :<br>TEXT PUBLISHED :<br>LE PASSAGE SUIVANT : | | | | LAUTET BERICHTIGT:<br>SHOULD READ :<br>DEVRAIT ETRE LU : |
|---|---|---|---|---|
| BET-Oberflächen von 0,03 bis<br>$m^2$/g beschrieben | 2 | 1 | 24/25 | BET-Oberflächen von 0,03 bis<br>$2m^2$/g und Porenvolumina von<br>0,25 bis 0,65 $cm^3$/g als<br>besonders geeignet beschrieben. |
| öchen 80,50 0,51 | 3 | 4 | 29 | kochen 0,50 0,51 |
| ät (%) (°C) tät (%) (°C) | 4 | 5 | 27 | tät (%) (%) tät (%) (%) |

| Tag der Entscheidung )<br>über die Berichtigung )<br>Date of decision on )<br>rectification: )<br>Date de décision portant ) | 3.2.89 | Ausgabe- und Ver- )<br>öffentlichungstag: )<br>Issue and publication )<br>date: )<br>Date d'edition et de ) | 23.8.89 | Patbl.Nr.)<br>) 89/34<br>·EPB no:) |
|---|---|---|---|---|

**Europäisches Patentamt**

**European Patent Office**

**Office européen des brevets**

(19)

(11) Veröffentlichungsnummer: **0 211 397**
**B1**

(12)

# EUROPÄISCHE PATENTSCHRIFT

(45) Veröffentlichungstag der Patentschrift:
26.10.88

(21) Anmeldenummer: 86110546.8

(22) Anmeldetag: 30.07.86

(51) Int. Cl.⁴: **B 01 J 23/66,** B 01 J 35/10,
C 07 D 301/10

(54) Silberkatalysator, seine Herstellung und Verwendung.

(30) Priorität: 07.08.85 DE 3528313

(43) Veröffentlichungstag der Anmeldung:
25.02.87 Patentblatt 87/9

(45) Bekanntmachung des Hinweises auf die Patenterteilung:
26.10.88 Patentblatt 88/43

(84) Benannte Vertragsstaaten:
AT BE CH DE FR GB LI NL

(56) Entgegenhaltungen:
EP - A - 0 097 935
EP - A - 0 161 930
FR - A - 2 117 183
FR - A - 2 253 747

(73) Patentinhaber: BASF Aktiengesellschaft,
Carl-Bosch-Strasse 38, D-6700 Ludwigshafen (DE)

(72) Erfinder: Boehning, Karl-Heinz, Dr.,
Walther-Rathenau-Strasse 32, D-6100 Darmstadt (DE)
Erfinder: Mross, Wolf Dieter, Dr.,
Anselm-Feuerbach-Strasse 21, D-6700 Frakenthal (DE)
Erfinder: Schwarzmann, Matthias, Dr.,
Carl-Bosch-Strasse 54, D-6703 Limburgerhof (DE)
Erfinder: Becker, Hans-Juergen, Dr.,
Bergsteinstrasse 36, D-6730 Neustadt (DE)
Erfinder: Plueckhan, Juergen, Dr., Bensheimer Ring 19 b,
D-6710 Frankenthal (DE)
Erfinder: Renner, Klaus-Christian, Dr.,
Carl-Spitzweg-Strasse 2, D-6710 Frankenthal (DE)

## Beschreibung

Es ist bekannt, Silber enthaltende Katalysatoren für die Direktoxidation von Ethylen mit Sauerstoff zu Ethylenoxid zu verwenden. Zumeist dient α-Aluminiumoxid als Träger für die Herstellung der Silberkatalysatoren. Es sind verschiedene Verfahren für die Aufbringung der katalytisch aktiven Komponente Silber auf den Träger bekannt (US-A 2 294 383, 3 423 328, 3 172 893).

Die Angaben über die wünschenswerten Eigenschaften, die das Trägermaterial aufweisen soll, gehen in der Literatur auseinander. In aller Regel werden deshalb in Patentschriften recht weite Bereiche für die physikalischen Eigenschaften des Trägers beansprucht.

So werden in DE-A 3 010 533 Oberflächen von weniger als 1 $m^2$/g und Porenvolumina von 0,2 bis 0,6 $cm^3$/g bei einem Porendurchmesser zwischen 500 und 50 000 nm angegeben. Die chemische Zusammensetzung des Trägers wird als nicht entscheidend bezeichnet. Als mögliches Schüttgewicht werden Werte von 700 kg/$m^3$ angegeben.

In DE-A 2 712 785 werden Träger mit BET-Oberflächen von 0,03 bis 2 $m^2$/g beschrieben. Zu ähnlichen Aussagen kommt die US-A 3 962 136.

Im Gegensatz dazu zeigen die jüngsten technischen Erfahrungen, dass nur kleine Ausschnitte aus den bekannten Bereichen wirklich vorteilhafte Eigenschaften bei den gefertigten Katalysatoren ergeben. Überraschend gute Ergebnisse bei Aktivität, Selektivität und vor allem Standzeit erzielt man bei der Direktoxidation von Ethylen mit Sauerstoff zu Ethylenoxid mit einem Silberkatalysator auf porösem Träger aus α-Aluminiumoxid, der bestimmte Mengen an löslichen Calcium-, Aluminium-, Kalium- und Natriumsalzen enthält, mit einer BET-Oberfläche zwischen 0,2 und 0,8 $m^2$/g, einem Porenvolumen von wenigstens 0,5 ml/g, wobei die Poren gleichermassen für kaltes und warmes Wasser zugänglich sind, einem Schüttgewicht unter 650 kg/$m^3$, und der eine Form hat, die im Reaktor eine geometrische Oberfläche von mindestens 600 $m^2$/$m^3$ realisiert, auf welcher mehr als 13 Gew.-% Silber als aktive Komponente aufgebracht werden und je $m^3$ Reaktor mehr als 110 kg Silber zur Verfügung stehen.

Der für die Wirksamkeit der Katalysatoren wichtige Träger wird aus Aluminiumoxid gefertigt. Sein Gehalt an α-$Al_2O_3$ soll mindestens 99 Gew.-% betragen. Die Gehalte an salpetersäurelöslichen Calcium- und/oder Aluminiumionenverbindungen können zwischen 200 und 2000 ppm liegen und die Gehalte an löslichen Kalium- und/oder Natriumverbindungen sollen 50 ppm nicht unterschreiten. Das Trägermaterial aus hochreinem α-$Al_2O_3$ soll zweckmässig eine BET-Oberfläche von nicht weniger als 0,3 $m^2$/g und nicht mehr als 0,7 $m^2$/g aufweisen. Das Porenvolumen des Trägermaterials wird zweckmässig durch die allgemein bekannte Methode der Wasseraufnahme bzw. der Quecksilberporosimetrie bestimmt. Das durch nur fünfminütiges Einwirken von Wasser bei Raumtemperatur festgestellte Porenvolumen soll sich nur unwesentlich, z.B. maximal 10%, von dem nach den genannten Methoden bestimmten Volumen unterscheiden.

Die Porenverteilung ist bimodal, wobei die grösseren Poren vorzugsweise zumindest 50% des Gesamtporenvolumens ergeben und einen mittleren Durchmesser von etwa 10 000 bis 40 000 nm haben, die kleineren Poren Durchmesser von etwa 500 bis 2000 nm. Man kann durch Auswahl geeigneter geometrischer Formen der Trägerpartikel das Schüttgewicht des Trägermaterials so einstellen, dass es 650 kg/$m^3$ nicht überschreitet und die äussere geometrische Oberfläche des Katalysators pro Reaktorvolumen dabei 600 $m^2$/$m^3$ nicht unterschreitet.

Die katalytisch aktiven Metallkomponenten werden durch ein Tränkverfahren aufgebracht, das aus einer oder mehreren Tränkstufen bestehen kann und das eine oder mehrere Temperungsstufen umfasst. Dabei soll der Katalysatorträger mit mindestens 13 Gew.-% Silber beaufschlagt werden und eine Silbermenge von mindestens 110 kg Silber pro $m^3$ Reaktor erreicht werden.

Die erfindungsgemässen Silberkatalysatoren werden für die Herstellung von Ethylenoxid aus Ethylen und Sauerstoff in der Gasphase eingesetzt. Der Vorteil besteht darin, dass diese beim Einsatz für die Herstellung von Ethylenoxid einen nur halb so grossen Selektivitätsabfall zeigen wie die bekannten Katalysatoren, die Standzeiten demzufolge deutlich höher sind. Gleichzeitig erhöht sich die Belastungsflexibilität der Katalysatoren.

*Beispiele, Katalysatorherstellung*

Die Herstellung der erfindungsgemässen und der nicht erfindungsgemässen Katalysatoren auf den verschiedenen Trägern wurde nach folgender allgemeiner Vorgehensweise durchgeführt:

Silbernitrat wird in zweimal soviel Molen sec.-Butylamin gelöst. Dieser Lösung werden je Ansatz für 100 g Träger 2 ml einer wässrigen Lithiumnitrat-Lösung (22,75 g $LiNO_3$ auf 100 ml Lösung) zugesetzt. Danach wird durch Zugabe von VE-Wasser das Lösungsvolumen auf das 1,1fache erhöht.

Entsprechend der zu erwartenden Flüssigkeitsaufnahme des Trägers wird dieser mit der Lösung imprägniert und einen Tag bei Raumtemperatur gelagert. Dann wird der imprägnierte Träger in einen Umluftofen überführt und bei 240 °C im Stickstoffstrom getempert, bis die Reaktion abgeklungen ist.

In einem zweiten Tränkschritt wird der Katalysator mit einer zur vollständigen Tränkung ausreichenden Menge einer methanolischen Lösung behandelt, die 1 Vol.-% sec.-Butylamin und 0,3 ml einer Caesiumhydroxid-Lösung (5,46 g CsOH auf 100 ml Lösung mit Methanol) enthält. Es schliesst sich ein Trocknungsschritt im Stickstoffstrom an.

*Anwendungsbeispiel 1*

Die obengenannten Katalysatoren werden zerkleinert und jeweils 10 g der Siebfraktion 0,6 bis 0,75 mm werden in einen Reaktor aus rostfreiem Stahl von 5 mm Innendurchmesser gefüllt. Der Reaktor besitzt einen Mantel, durch den eine thermostatisierende Flüssigkeit geschickt wird. Durch den Reaktor wird ein Gas der Zusammensetzung: 30% Ethylen, 8% Sauerstoff, 2 ppm Inhibitor, Rest Stickstoff geleitet. Der Druck beträgt 16 bar und die Katalysator-

belastung 3300 Nl Gas/l Kontakt × h. Die Temperatur wird so eingeregelt, dass der Sauerstoffumsatz 50% beträgt. Nach 2 Tagen werden Proben gezogen und Aktivität und Selektivität bestimmt.

Die vorteilhaften reaktionstechnischen Ergebnisse, wie sie sich mit den auf den Trägern A und B hergestellten Katalysatoren erzielen lassen, sind in Tabelle 3 aus Mittelwerten verschiedener Messungen zusammengestellt. Die weniger günstigen Ergebnisse mit allen anderen Trägermaterialien (C-I) zeigt der zweite Teil der Tabelle.

*Anwendungsbeispiel 2*

Die Katalysatoren A, B, C, F und I werden unzerkleinert in einer Menge von je 13 dm³ in einen stählernen druckfesten Rohrreaktor gefüllt, der dem in technischen Anlagen üblichen Einzelrohr entspricht. Der Reaktor besitzt einen Mantel, durch den eine thermostatisierende Flüssigkeit geschickt wird. Durch den Reaktor wird ein Gas der ungefähren Zusammensetzung: 30% Ethylen, 8% Sauerstoff, 6,5% CO₂, 4% Argon, 3 ppm Inhibitor und 50% Methan geschickt. Der Druck beträgt 16 bar. Die Temperatur im Kühlmedium des Reaktors wird in einer ersten Versuchsreihe a so eingestellt, dass bei einer Raumgeschwindigkeit von 2000 Nm³ Gas/m³ Kontakt × h ein Sauerstoffumsatz von 35% erreicht wird. In einer zweiten Versuchsreihe wird bei einer Raumgeschwindigkeit von 4000 Nm³ Gas/m³ Kontakt × h 50% des Sauerstoffs umgesetzt. Proben werden jeweils nach einem Monat und nach 12 Monaten gezogen.

Tabelle 4 zeigt Standzeitverhalten und Belastungsflexibilität der eingesetzten Katalysatoren.

Die erfindungsgemässen Träger haben die in der nachfolgenden Tabelle 1 zusammengestellten Eigenschaften. Vergleichbare bekannte Träger mit in verschiedener Hinsicht abweichenden Eigenschaften sind in Tabelle 2 enthalten. Diese Träger haben nicht die gewünschten vorteilhaften Eigenschaften, wie in den folgenden Beispielen gezeigt wird.

### TABELLE 1

#### Physikalische und chemische Daten der erfindungsgemässen Träger

| Trägerbezeichnung | | | A | B |
|---|---|---|---|---|
| Gehalt an α-Al₂O₃ (Gew.-%) | | | 99,8 | 99,5 |
| Gehalt an SiO₂ | (Gew.-%) | | 0,1 | 0,4 |
| Gehalt an mit HNO₃ löslichen Ionen | (Gew.ppm) | | | |
| | Al | | 300-2000 | 600-2000 |
| | Ca | | 300-2000 | 500-2000 |
| | K | | 50-1000 | 50-1000 |
| | Na | | 50-1000 | 80-1000 |
| BET-Oberfläche | (m²/g) | | 0,60 | 0,60 |
| Wasseraufnahme | (ml/g) | | | |
| | kalt | | 0,45 | 0,46 |
| | kochen | | 80,50 | 0,51 |
| Schüttgewicht | (kg/m³) | | 620 | 600 |
| spez. Oberfläche | (m²/m³) | | 640 | 670 |
| Silberdichte | (kg/m³) | | 115 | 115 |

### TABELLE 2

#### Eigenschaften von nicht erfindungsgemässen Trägermaterialien

| Trägerbezeichnung | | C | D | E | F | G | H | I |
|---|---|---|---|---|---|---|---|---|
| Gehalt an α-Al₂O₃ (Gew.-%) | | 99,8 | 99,5 | 99,8 | 99,8 | 99,8 | 98,5 | 99,6 |
| Gehalt an SiO₂ | (Gew.-%) | 0,1 | 0,4 | 0,1 | 0,05 | 0,04 | 1,4 | 0,1 |
| Gehalt an mit HNO₃ löslichen Ionen | (Gew.ppm) | | | | | | | |
| | Al | 250 | 600 | 300 | 100 | 150 | 60 | 130 |
| | Ca | 350 | 500 | 400 | 100 | 100 | 10 | 300 |
| | K | 20 | 40 | 30 | 5 | 5 | 2 | 10 |
| | Na | 30 | 80 | 80 | 60 | 100 | 50 | 20 |
| BET-Oberfläche | (m²/g) | 0,45 | 0,50 | 0,50 | 0,35 | 0,35 | 0,35 | 0,25 |
| Wasseraufnahme | (ml/g) | | | | | | | |
| | kalt | 0,34 | 0,33 | 0,35 | 0,39 | 0,28 | 0,31 | 0,22 |
| | kochen | 0,43 | 0,42 | 0,43 | 0,48 | 0,35 | 0,35 | 0,25 |
| Schüttgewicht | (kg/m³) | 730 | 730 | 600 | 650 | 810 | 810 | 1050 |
| geometrische Oberfläche | (m²/m³) | 670 | 670 | 570 | 630 | 630 | 630 | 670 |
| Silberdichte | (kg/m³) | 100 | 100 | 90 | 105 | 110 | 110 | 85 |

## TABELLE 3

Ergebnisse der Versuche nach
Anwendungsbeispiel 1

| Träger | Selektivität (%) | Aktivität (°C) |
|--------|------------------|----------------|
| A | 82,3 | 219 |
| B | 82,2 | 220 |
| C | 81,9 | 222 |
| D | 81,9 | 225 |
| E | 81,5 | 227 |
| F | 81,0 | 221 |
| G | 80,9 | 224 |
| H | 80,1 | 230 |
| I | 80,0 | 226 |

## TABELLE 4

Ergebnisse der Versuche nach
Anwendungsbeispiel 2

| Träger | Selektivität (%) | Aktivität (°C) | Selektivität (%) | Aktivität (°C) |
|--------|------------------|----------------|------------------|----------------|
| | bei 35% $O_2$-Umsatz | | bei 50% $O_2$-Umsatz | |
| nach 1 Monat | | | | |
| A | 82,5 | 196 | 81,5 | 220 |
| B | 82,5 | 197 | 81,3 | 220 |
| C | 82,1 | 200 | 80,4 | 226 |
| F | 81,5 | 200 | 80,0 | 228 |
| I | 81,0 | 204 | 79,5 | 234 |
| nach 12 Monaten | | | | |
| A | 81,5 | 200 | 80,4 | 228 |
| B | 81,6 | 205 | 80,4 | 230 |
| C | 80,5 | 210 | 79,0 | 238 |
| F | 80,2 | 208 | 78,4 | 242 |
| I | 79,4 | 213 | 77,0 | 249 |

## Patentansprüche

1. Silberkatalysator für die Direktoxidation von Ethylen mit Sauerstoff zu Ethylenoxid auf einem porösen Träger, der im wesentlichen aus α-Aluminiumoxid besteht und bestimmte Mengen an löslichen Calcium-, Aluminium-, Kalium- und Natriumsalzen enthält, dadurch gekennzeichnet, dass der Träger eine BET-Oberfläche zwischen 0,2 und 0,8 m²/g, ein Porenvolumen von wenigstens 0,5 ml/g, wobei die Poren gleichermassen für kaltes und warmes Wasser zugänglich sind, ein Schüttgewicht unter 650 kg/m³ und eine Form hat, die im Reaktor eine geometrische Oberfläche von mindestens 600 m²/m³ realisiert, auf welcher mehr als 13 Gew.-% Silber als aktive Komponente aufgebracht werden und je m³ Reaktor mehr als 110 kg Silber zur Verfügung stehen.

2. Silberkatalysator nach Anspruch 1, dadurch gekennzeichnet, dass im Träger salpetersäurelösliche Calcium- und/oder Aluminiumverbindungen in Mengen von 200 bis 2000 ppm und mehr als 50 ppm lösliche Kalium- und/oder Natriumverbindungen enthalten sind.

3. Silberkatalysator nach Anspruch 1 und 2, dadurch gekennzeichnet, dass das Trägermaterial zu mehr als 99% aus hochreinem α-Al₂O₃ besteht und eine BET-Oberfläche von nicht weniger als 0,3 m²/g und nicht mehr als 0,7 m²/g aufweist.

4. Silberkatalysator nach Anspruch 1 bis 3, dadurch gekennzeichnet, dass das Trägermaterial ein Porenvolumen von wenigstens 0,5 cm³/g besitzt.

5. Silberkatalysator nach Anspruch 1 bis 4, dadurch gekennzeichnet, dass die Porenverteilung bimodal ist, wobei die grösseren Poren wenigstens 50% des Gesamtporenvolumens betragen und einen mittleren Durchmesser von 10 000 bis 40 000 und die kleineren Poren einen Durchmesser von 500 bis 2000 nm haben.

6. Verfahren zur Herstellung eines Silberkatalysators nach Anspruch 1 bis 5, dadurch gekennzeichnet, dass man als Träger α-Al₂O₃ verwendet und die katalytisch aktive Masse durch Tränken desselben in einer oder mehreren Stufen mit einer Silbersalzlösung, die komplexbildende Zusätze und Alkalisalze enthält, aufgebracht und ebenso in einer oder mehreren Stufen getempert ist, wobei der Katalysatorträger mit mindestens 13 Gew.-% Silber beaufschlagt und eine Silbermasse von mindestens 110 kg Silber pro m³ Reaktor erreicht wird.

7. Verwendung der Silberkatalysatoren nach Anspruch 1 bis 5 für die Herstellung von Ethylenoxid aus Ethylen und Sauerstoff in der Gasphase.

## Claims

1. A silver catalyst for the direct oxidation of ethylene with oxygen to give ethylene oxide, on a porous carrier which essentially consists of α-alumina and contains certain amounts of soluble calcium, aluminium, potassium and sodium salts, wherein the carrier has a BET surface area of from 0.3 to 0.8 m²/g, a pore volume of not less than 0.5 ml/g, the pores being equally accessible to cold and warm water, and a bulk density of less than 650 kg/m³, and has a shape which, in the reactor, provides a geometric surface area of not less than 600 m²/m³, on which more than 13% by weight of silver are applied as the active component, more than 110 kg of silver being available per m³ of reactor.

2. A silver catalyst as claimed in claim 1, wherein the carrier contains from 200 to 2000 ppm of calcium and/or aluminium compounds which are soluble in nitric acid and more than 50 ppm of soluble potassium and/or compounds.

3. A silver catalyst as claimed in claim 1 or 2, wherein the carrier consists of more than 99% of very pure α-Al₂O₃ and has a BET surface area of not less than 0.3 m²/g and not more than 0.7 m²/g.

4. A silver catalyst as claimed in claim 1 or 2 or 3, wherein the carrier has a pore volume of not less than 0.5 cm³/g.

5. A silver catalyst as claimed in claim 1 or 2 or 3 or 4, wherein the pore distribution is bimodal, the larger pores accounting for not less than 50% of the total pore volume and having a mean diameter of from 10 000 to 40 000 nm, and the smaller pores having a diameter of from 500 to 2000 nm.

6. A process for the preparation of a silver catalyst as claimed in claim 1 or 2, or 3 or 4 or 5, wherein $\alpha\text{-Al}_2\text{-O}_3$ is used as the carrier, and the catalystically active material is applied by impregnating the carrier, in one or more stages, with a silver salt solution which contains complex-forming additives and alkali metal salts, and is also heated in one or more stages, not less than 13% by weight or silver being deposited on the catalyst carrier and an amount of silver corresponding to not than 110 kg per m³ of reactor being achieved.

7. Use of á silver catalyst as claimed in claim 1 or 2 or 3 or 4 or 5 for the preparation of ethylene oxide from ethylene and oxygen in the gas phase.

## Revendications

1. Catalyseur à l'argent pour l'oxydation directe par l'oxygène de l'éthylène en oxyde d'éthylène sur un support poreux, constitué essentiellement d'oxyde d'aluminium α et contenant des proportions déterminées de sels de calcium, d'aluminium, de potassium et de sodium solubles, caractérisé en ce que le support possède une surface selon BET comprise entre 0,2 et 0,8 m²/g, un volume de pores également accessibles à l'eau froide et à l'eau chaude d'au moins 0,5 ml/g, une masse volumique apparente inférieure à 650 kg/m³ et une forme permettant de réaliser dans le réacteur une surface géométrique au moins égale à 600 m²/m³, sur laquelle sont déposés plus de 13% en poids d'argent comme composant catalytique actif, permettant de disposer de plus de 110 kg d'argent par m³ de réacteur.

2. Catalyseur à l'argent suivant la revendication 1, caractérisé en ce que le support contient entre 200 et 2000 ppm de composés du calcium et(ou) de l'aluminium solubles dans l'acide nitrique et une proportion supérieure à 50 ppm de composés solubles du potassium et(ou) du sodium.

3. Catalyseur à l'argent suivant la revendication 1 et la revendication 2, caractérisé en ce que le support est constitué à plus de 99% de $\text{Al}_2\text{O}_3$ α très pur et possède une surface selon BET au moins égale à 0,3 m²/g et au plus égale à 0,7 m²/g.

4. Catalyseur à l'argent suivant les revendications 1 à 3, caractérisé en ce que le support possède un volume de pores au moins égal à 0,5 cm³/g.

5. Catalyseur à l'argent suivant les revendications 1 à 4, caractérisé en ce que les pores possèdent une répartition bimodale, les pores plus grands, constituant au moins 50% du volume total des pores, possèdant un diamètre moyen compris entre 10 000 et 40 000 nm et les pores plus petits un diamètre allant de 500 à 2000 nm.

6. Procédé de préparation d'un catalyseur à l'argent suivant les revendications 1 à 5, caractérisé en ce que l'on emploie comme support du $\text{Al}_2\text{O}_3$ α, sur lequel la matière catalytique active est déposée par un ou plusieurs stades d'imprégnation avec une solution d'un sel d'argent, contenant des additifs complexants et des sels de métaux alcalins, puis soumise à un ou plusieurs stades de traitement thermique, le support du catalyseur portant au moins 13% en poids d'argent et permettant d'atteindre une quantité d'argent au moins égale à 110 kg d'argent per m³ de réacteur.

7. Utilisation des catalyseurs à l'argent suivant les revendications 1 à 5 pour la production d'oxyde d'éthylène dans la phase gazeuse à partir d'éthylène et d'oxygène.